(19) Europäisches Patentamt
European Patent Office
Office européen des brevets

(11) EP 1 082 954 A2

# (12) EUROPÄISCHE PATENTANMELDUNG

(43) Veröffentlichungstag:
**14.03.2001 Patentblatt 2001/11**

(51) Int. Cl.[7]: **A61K 7/42**

(21) Anmeldenummer: **00118243.5**

(22) Anmeldetag: **04.09.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **08.09.1999 DE 19942714**

(71) Anmelder:
**Beiersdorf Aktiengesellschaft**
**20245 Hamburg (DE)**

(72) Erfinder:
- **Gers-Barlag, Heinrich, Dr.**
  **25495 Kummersfeld (DE)**
- **Müller, Anja**
  **23843 Rümpel (DE)**
- **Grundt, Wiebke**
  **21244 Buchholz (DE)**

## (54) Kosmetische Lichtschutzzubereitungen enthaltend ein oder mehrere unter den Amidverbindungen ausgewählte Öle und ein oder mehrere unsymmetrisch substituierte Triazinderivate

(57) Kosmetische Lichtschutzzubereitungen, enthaltend ein oder mehrere aus den Amidverbindungen ausgewählte Öle und ein oder mehrere unsymmetrisch substituierte Triazinderivate.

EP 1 082 954 A2

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der symmetrisch substituierte 4,4',4''-(1,3,5-Triazin-2,4,6-triyltriimino)-trisbenzoesäure-tris(2-ethylhexylester), synonym: 2,4,6-Tris-[anilino-(p-carbo-2'-ethyl-1'-hexyloxy)]-1,3,5-triazin.

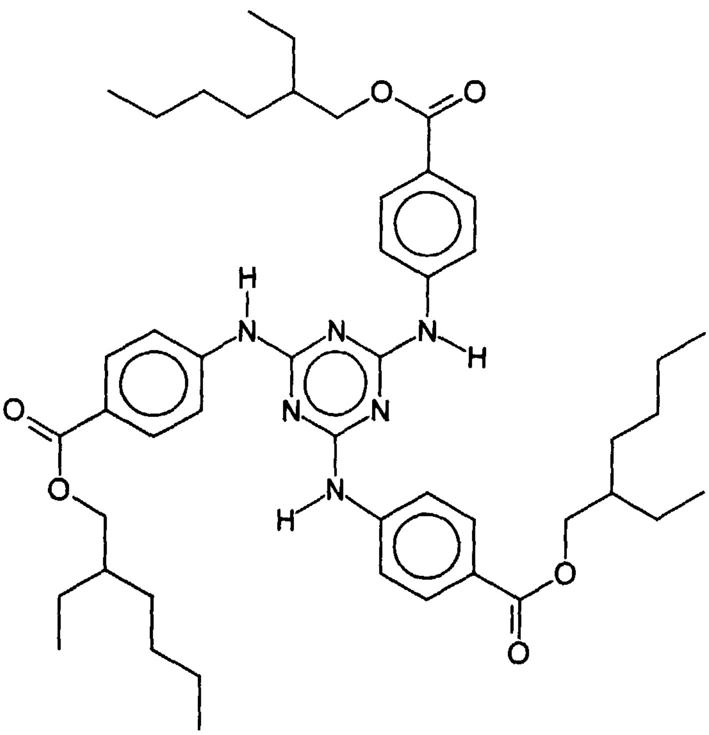

Diese UVB-Filtersubstanz wird von der BASE Aktiengesellschaft unter der Warenbezeichnung UVINUL® T 150 vertrieben und zeichnet sich durch gute UV-Absorptionseigenschaften aus. Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz. Zwar beschreibt die DE-OS 196 33 012 die Verwendung von Campherderivaten, zur Erzielung oder Erhöhung der Löslichkeit von Triazinderivaten in Ölkomponenten. Als besonders vorteilhaft herausgestellt werden dabei insbesondere der 4-Methylbenzylidencampher, welcher eine vorzügliche Lichtschutzfiltersubstanz darstellt, die sich durch die Struktur

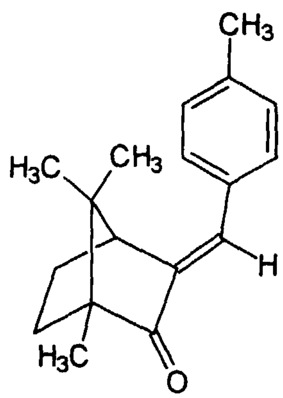

auszeichnet und von Merck unter der Marke Eusolex® 6300 verkauft wird.

Als vorteilhaft wird ferner herausgestellt der Benzylidencampher, welcher sich durch die Struktur

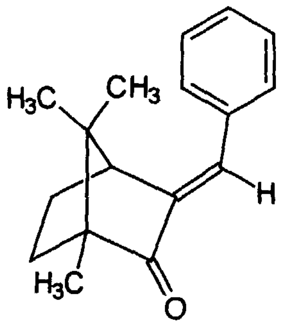

auszeichnet und von der Gesellschaft Induchem unter der Marke Unisol® S22 verkauft wird.

Ferner beschreibt die DE-OS 196 35 057 die Verwendung von Dibenzoylmethanderivaten zur Erzielung oder Erhöhung der Löslichkeit von Triazinderivaten in Ölkomponenten. Als besonders vorteilhaft werden dabei das 1-(4'-Isopropylphenyl)-3-phenyl-1-propan-1,3-dion (nach INCI: Isopropyldibenzoylmethan) herausgestellt, welches sich durch die Struktur

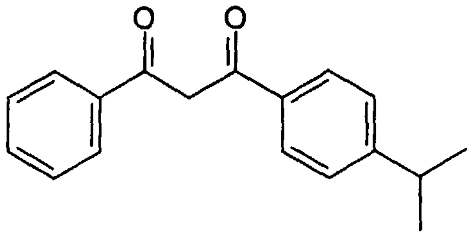

auszeichnet und von Merck unter der Marke Eusolex® 8020 verkauft wird, sowie das 1-(4'-t-Butylphenyl)-3-(4-methoxyphenyl)-1-propan-1,3-dion (nach INCI: Butylmethoxydibenzoylmethan), welches sich durch die Struktur

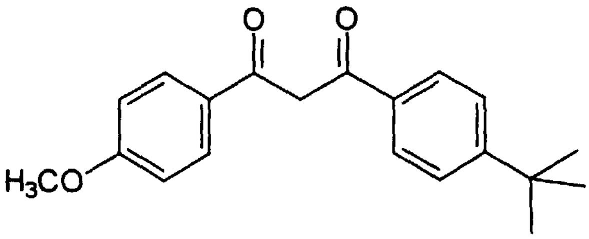

auszeichnet und von der Gesellschaft Givaudan unter der Marke Parsol® 1789 verkauft wird.

Dennoch bestand der Nachteil des Standes der Technik, daß in der Regel entweder nur vergleichsweise niedrige Lichtschutzfaktoren erreicht werden konnten, oder daß die Lichtschutzfilter nicht die genügende UV-Stabilität aufwiesen oder nicht genügende physiologische Verträglichkeit aufwiesen oder nicht genügend hohe Löslichkeit oder Dispergierbarkeit in kosmetischen oder dermatologischen Zubereitungen aufwiesen oder auch sonstige Inkompatibilitäten mit kosmetischen oder dermatologischen Zubereitungen oder mehrere Nachteile zugleich.

Von verschiedenen Autoren wurden UV-Filtersubstanzen vorgestellt, welche das Strukturmotiv

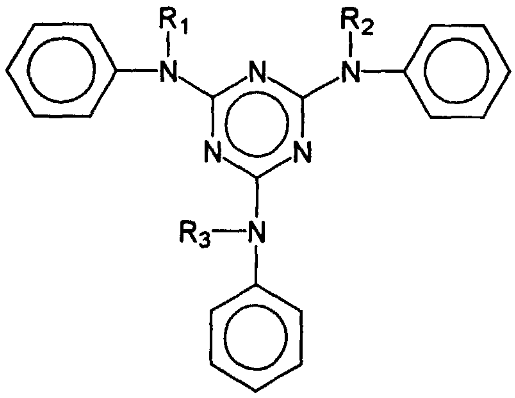

aufweisen.

Hinsichtlich der $C_3$-Achse des Triazingrundkörpers sind sowohl symmetrische Substitution wie auch unsymmetrische Substitution denkbar. In diesem Sinne symmetrisch substituierte s-Triazine weisen drei gleiche Substituenten auf und werden beispielsweise vertreten durch den 4,4',4"-(1,3,5-Triazin-2,4,6-triyltriimino)-tris-benzoesäure-tris(2-ethylhexylester), analog der INCI-Nomenklatur auch: Octyltriazon, welcher durch folgende Struktur wiedergegeben wird:

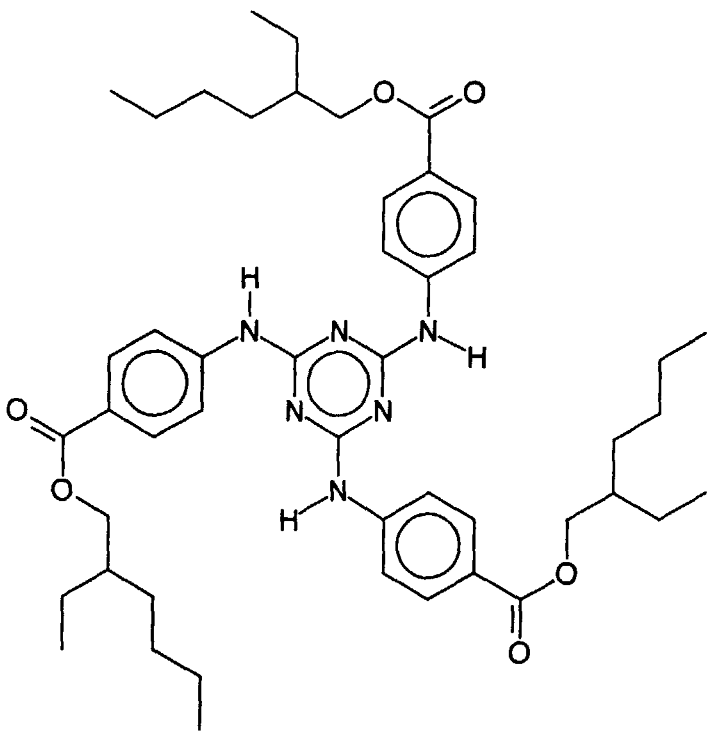

Hinsichtlich der $C_3$-Achse unsymmetrisch substituierte s-Triazinderivate weisen demzufolge unterschiedliche Substituenten auf, wodurch die $C_3$-Symmetrie zerstört wird. Im Sinne der hiermit vorliegenden Erfindung wird als „symmetrisch" bzw. „unsymmetrisch" stets symmetrisch bzw. unsymmetrisch hinsichtlich der $C_3$-Achse des Triazingrundkörpers verstanden, es sei denn, etwas Anderes wäre ausdrücklich erwähnt.

So werden in der EP-A- 570 838 unsymmetrisch substituierte s-Triazinderivate beschrieben, deren chemische Struktur durch die genetische Formel

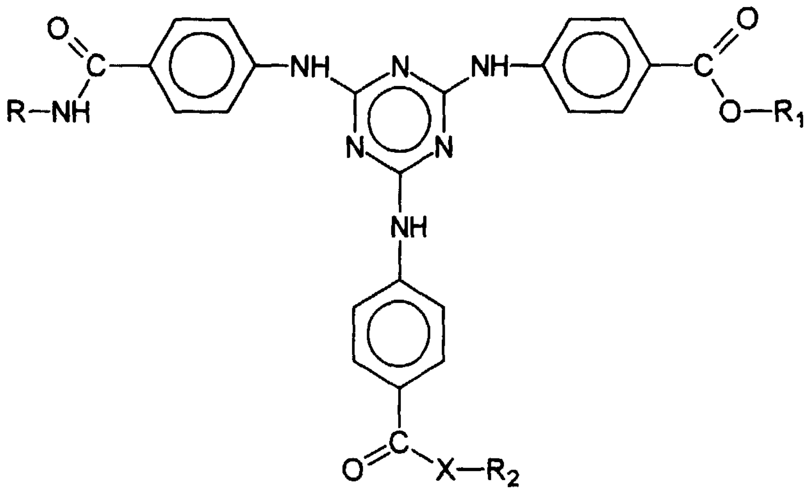

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

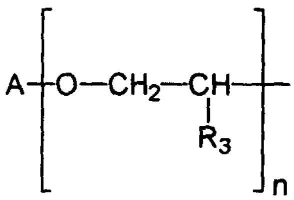

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

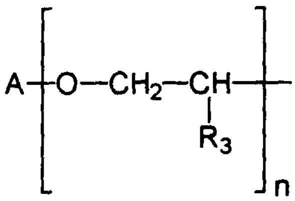

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

Solche s-Triazinderivate, die sich im Gegensatze zum 4,4',4''-(1,3,5-Triazin-2,4,6-triyltriimino)-tris-benzoesäure-tris(2-ethylhexylester) durch verbesserte Löslichkeit in vielen Ölkomponenten auszeichnen, können gemäß der Lehre der Schriften EP-A-821 937, EP-A-821 938, EP-A-821 939, EP-A-821 940 und EP-A-821 941 mit verschiedenen anderen Lichtschutzfiltern in kosmetischen oder dermatologischen Zubereitungen kombiniert werden, wodurch bestimmten technischen Sachverhalten Rechnung getragen werden soll.

Auch andere als Festkörper vorliegende UV-Filtersubstanzen, deren Einarbeitung in kosmetische oder dermatologische Lichtschutzformulierungen zumindest gewisse Probleme aufweist, sind bekannt. So werden in der EP-A-775 698 Bis-Resorcinyltriazinderivate beschrieben, deren chemische Struktur durch die generische Formel

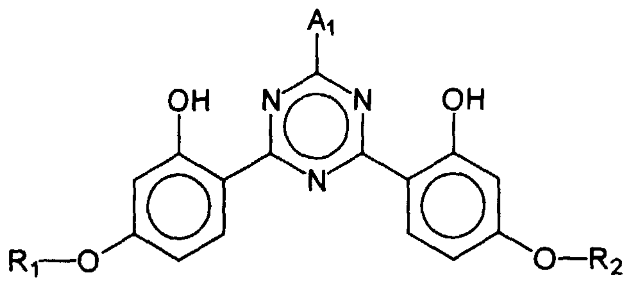

wiedergegeben wird, wobei $R_1$ , $R_2$ und $A_1$ verschiedenste organische Reste repräsentieren.

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Die teilweise vorstehend genannten Verbindungen, welche als Lichtschutzmittel für kosmetische und dermatologische Lichtschutzformulierungen eingesetzt werden, zeichnen sich, wie gesagt, an sich durch gute Lichtschutzwirkung aus. Sie haben jedoch den Nachteil, daß es bisweilen schwierig ist, sie in befriedigender Weise solchen Formulierungen einzuverleiben.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Homschicht der Haut verteilt ist.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solange gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte „Ozonloch" zum einen zuverlässige Angaben des Herstellers zum Lichtschutzfaktor, zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Wenigstens einigen, wenn nicht allen diesen Nachteilen abzuhelfen, war eine der Aufgaben der vorliegenden Erfindung.

Die Verwendung von Insektenrepellentien in kosmetischen Zubereitungen ist an sich bekannt. Repellentien sind Mittel, die abwehrend oder und vertreibend auf andere Lebewesen, insbesondere Schädlinge und Lästlinge, wirken. Viele der Mittel veranlassen durch ihren unangenehmen Geruch und Geschmack, daß die Tiere sich von z. B. Nahrung od. bestimmten Plätzen fernhalten. Hierzu gehören Mittel zur Abschreckung von Insekten u. Spinnen, zu denen auch Octansäurediethylamid und 3-(N-Acetyl-N-butylamino)-propionsäureethylester zählen, ferner manche Holzschutzmittel sowie Fraßhemmstoffe oder „Fraßschutzmitter, z. B. im Pflanzenschutz, für Saatgut oder für Textilien (Mittel zur Mottenbekämpfung, gegen Silberfischchen und Teppichkäfer).

Es war indes überraschend und für den Fachmann nicht vorauszusehen, daß kosmetische Lichtschutzzubereitungen, enthaltend ein oder mehrere aus den Amidverbindungen ausgewählte Öle und ein oder mehrere unsymmetrisch substituierte Triazinderivate, den Nachteilen des Standes der Technik abhelfen..

Zwar beschreibt die EP-A-0 748 623 kosmetische Zusammensetzungen zur topischen Anwendung, insbesondere zum Lichtschutz der Haut und/oder der Haare, dadurch gekennzeichnet, daß sie in einem kosmetisch akzeptablen Träger (i) 2,4,6-Tris[p-(2'-ethylhexyl-1'-oxycarbonyl-)-anilino]-1,3,5-triazin, also ein symmetrisch substituiertes Triazinderivat, als Lichtschutzfilter und (ii) mindestens ein unter den Amidverbindungen ausgewähltes Öl enthalten.

Diese Schrift konnte allerdings nicht den Weg zur vorliegenden Erfindung ebnen, da die Wirkung der unter den Amidverbindungen ausgewählten Öle auf symmetrisch substituierte Triazine überraschend weit hinter derjenigen auf unsymmetrisch substituierte zurückbleibt. Darüberhinaus gibt die EP-A-0 748 623 keinen Hinweis der insbesondere auf die verstärkte Lichtschutzleistung, die bei Befolgen der erfindungsgemäßen Lehre erzielt werden kann.

Eine vorteilhafte Ausführungsform der Erfindung ist die Verwendung eines oder mehrerer aus den Amidverbindungen ausgewählter Öle zur Erzielung oder der Erhöhung der Löslichkeit von einem oder mehreren unsymmetrisch substituierten s-Triazinderivaten in

(a) entweder einer isolierten Ölkomponente oder

(b) in mindestens einer Ölphase eines dispersen Zwei- oder Mehrphasensystem welches zusätzlich eine oder mehrere Wasserphasen enthalten kann,

Erfindungsgemäß ist ferner die die Verwendung eines oder mehrerer aus den Amidverbindungen ausgewählter Öle zur Erhöhung der UVA- und/oder der UVB-Schutzleistung von Lichtschutzzubereitungen, welche ein oder mehrere unsymmetrisch substituierte s-Triazinderivate enthalten.

Erfindungsgemäß bevorzugt ist insbesondere, wann das oder die aus den Amidverbindungen ausgewählten Öle gewählt werden aus der Gruppe der Substanzen der generischen Strukturformel

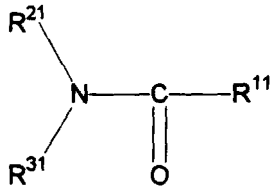

,

in welcher $R^{11}$, $R^{21}$ und $R^{31}$ unabhängig voneinander gewählt werden aus der Gruppe der verzweigten und unverzweigten, gesättigten und ungesättigten und gegebenenfalls funktional substituierten Alkylreste und/oder Cycloalkyreste und/oder gegebenenfalls funktional substituierten Arylreste, und wobei $R^{21}$ und/oder $R^{31}$ auch die Struktur

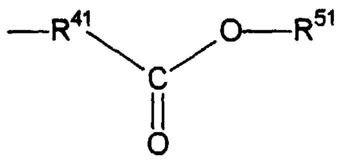

annehmen kann oder können, in welcher $R^{41}$ eine Alkylengruppe mit bis zu 10 Kohlenstoffatome bedeutet und $R^{51}$ gewählt wird aus der Gruppe der verzweigten und unverzweigten, gesättigten und ungesättigten und gegebenenfalls funktional substituierten Alkylreste.

Insbesondere vorteilhaft werden das oder die aus den Amidverbindungen ausgewählten Öle gewählt aus der Gruppe

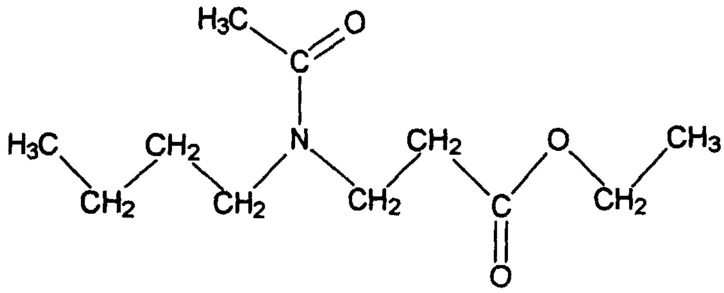

(3-(N-Acetyl-N-butylamino)-propionsäureethylester, = Repellent 3535)

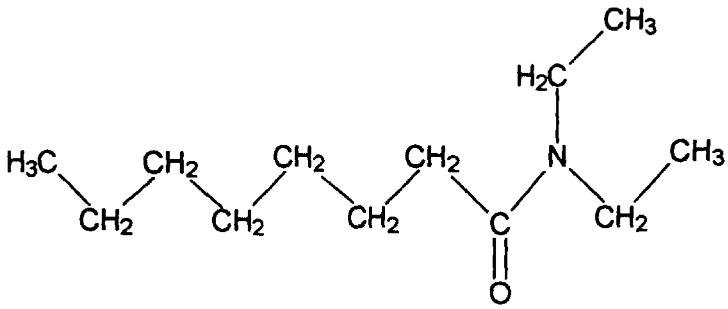

(Octansäurediethylamid = Repellent 790)

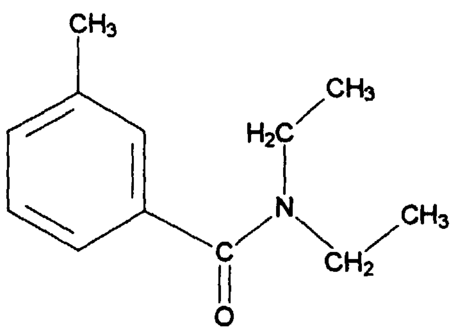

(N,N-Diethyl-m-toluamid = DEET)

Erfindungsgemäß ist ferner die die Verwendung einer oder mehrerer aus der Gruppe 3-(N-Acetyl-N-butylamino)-propionsäureethylester, Octansäurediethylamid, N,N-Diethyl-m-toluamid ausgewählter Substanzen zur Erzielung oder der Erhöhung der Löslichkeit eines oder mehrerer unsymmetrisch substituierter s-Triazinderivate in

(a) entweder einer isolierten Ölkomponente oder
(b) in mindestens einer Ölphase eines dispersen Zwei- oder Mehrphasensystem welches zusätzlich eine oder mehrere Wasserphasen enthalten kann,

Erfindungsgemäß ist darüberhinaus die die Verwendung einer oder mehrerer aus der Gruppe 3-(N-Acetyl-N-butylamino)-propionsäureethylester, Octansäurediethylamid, N,N-Diethyl-m-toluamid ausgewählter Substanzen zur Erhöhung der UVA- und/oder der UVB-Schutzleistung von Lichtschutzzubereitungen, welche ein oder mehrere unsymmetrisch substituierte s-Triazindenvate enthalten.

Die Gesamtmenge an einem oder mehreren aus den Amidverbindungen ausgewählten Öle in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen.

In besonders vorteilhaften Ausführungsformen werden das oder die unsymmetrisch substituierten s-Triazinderivate gewählt aus der Gruppe der Substanzen, deren chemische Struktur durch die generische Formel

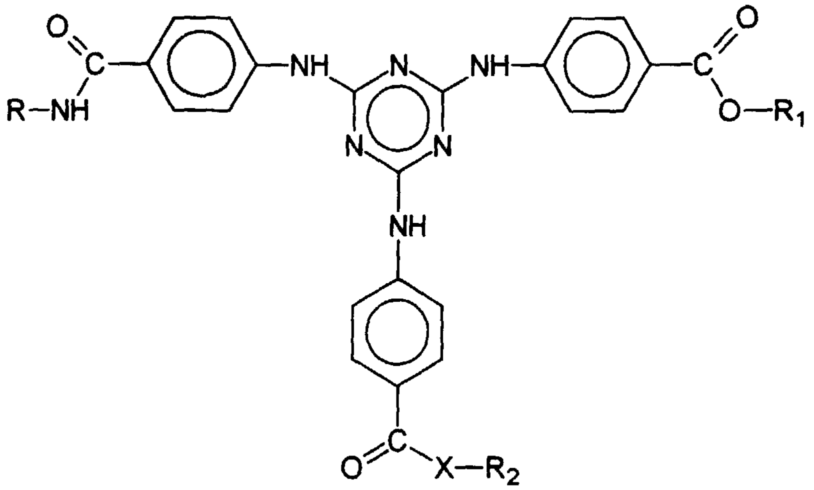

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
X ein Sauerstoffatom oder eine NH-Gruppe darstellt,
$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

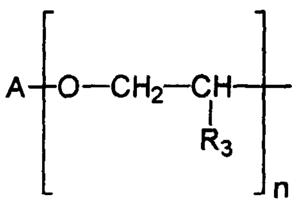

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

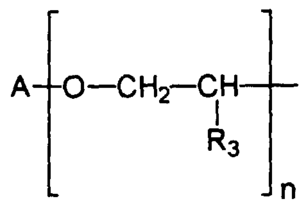

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

In besonders vorteilhaften Ausführungsformen werden das oder die unsymmetrisch substituierten s-Triazinderivate gewählt aus der Gruppe der Substanzen, welche in der EP-A-775 698 beschrieben werden:

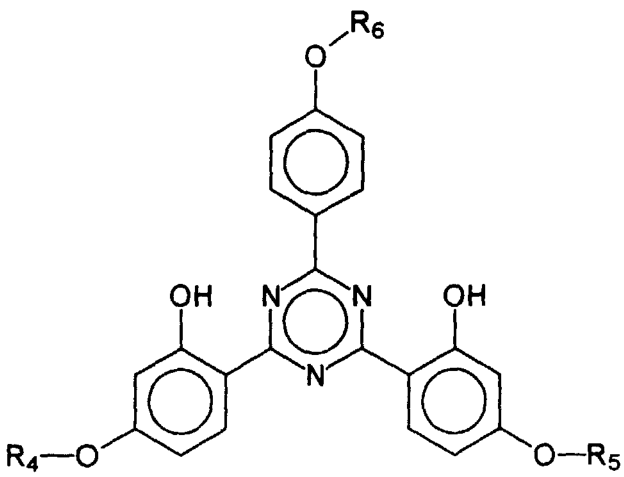

Alle in dieser Schrift erwähnten Bis-Resorcinyltriazine, seien sie durch generische Formeln offenbart, seien sie durch konkrete Formeln offenbart, sind vorteilhaft im Sinne der vorliegenden Erfindung zu verwenden.Ganz besonders vorteilhaft werden $R_4$ und $R_5$ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

$A_1$ stellt vorteilhaft einen substituierten homo- oder heterocycilschen Aromatischen Fünfring oder Sechsring dar.

Ganz besonders vorteilhaft sind folgende Verbindungen:

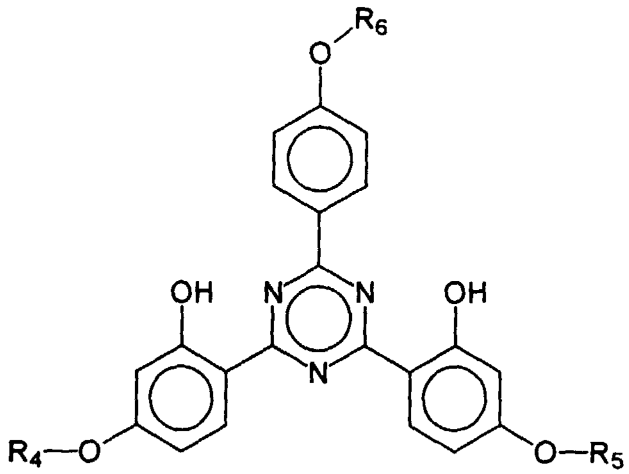

wobei $R_6$ ein Wasserstoffatom oder eine verzweigte oder unverzweigte Alkylgruppe mit 1 bis 10 Kohlenstoffatomen darstellt, insbesondere das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

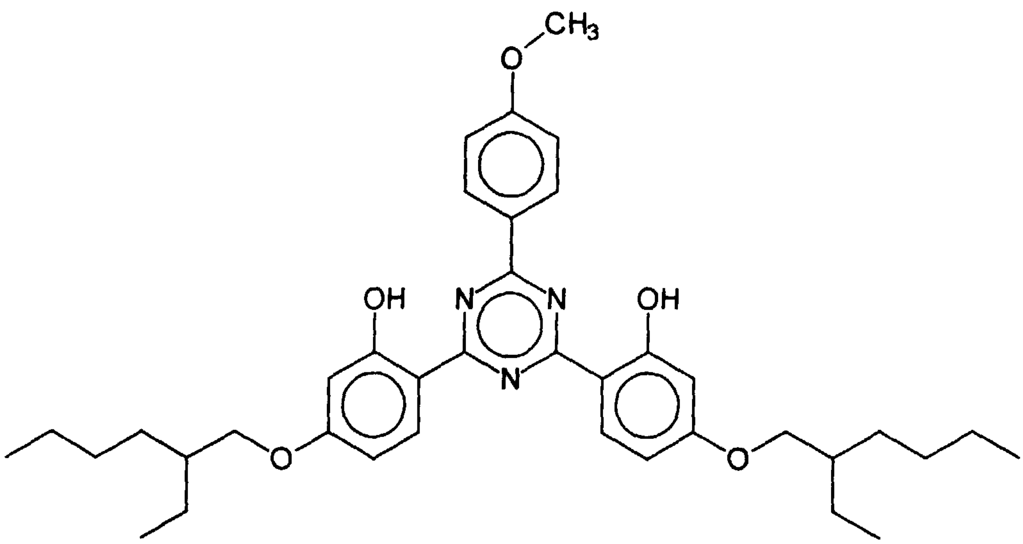

Ferner vorteilhaft ist das 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist:

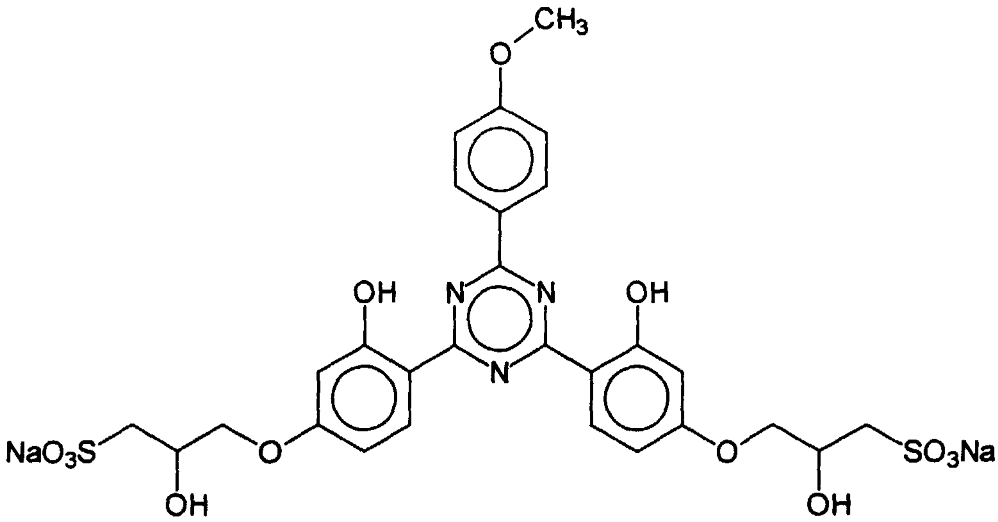

Ferner vorteilhaft ist das 2,4-Bis-{[4-(3-(2-Propyloxy)-2-hydroxy-propyloxy)-2-hydroxy]-phenyl}-6-(4-methoxyphenyl)-1,3,5-triazin (welches die INCI-Bezeichnung Aniso Triazin trägt), welches durch folgende Struktur gekennzeichnet ist:

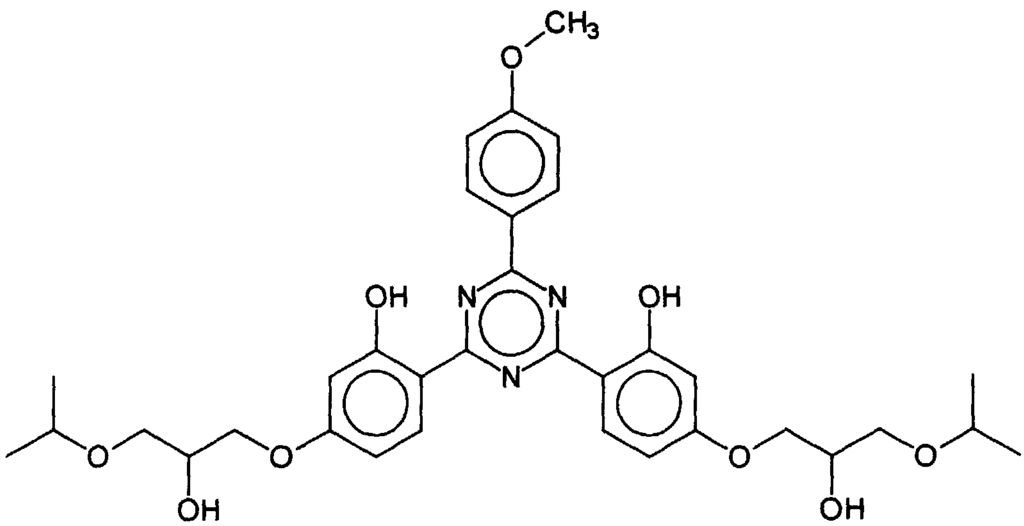

Ferner vorteilhaft ist das 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist:

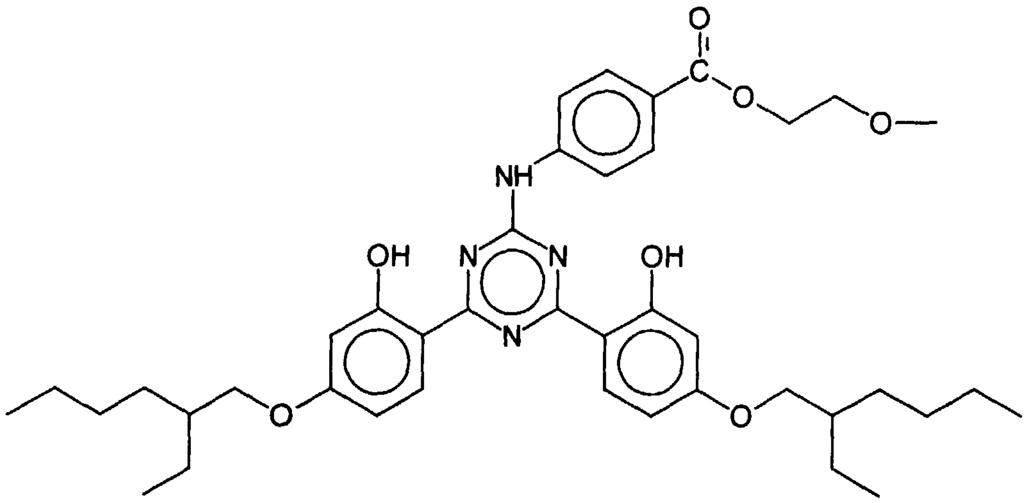

Ferner vorteilhaft ist das 2,4-Bis-{[4-(3-(2-propyloxy)-2-hydroxy-propyloxy)-2-hydroxy]-phenyl}-6-(4-(2-ethylcarboxyl)-phenylamino]-1,3,5-triazin, welches durch folgende Struktur gekennzeichnet ist:

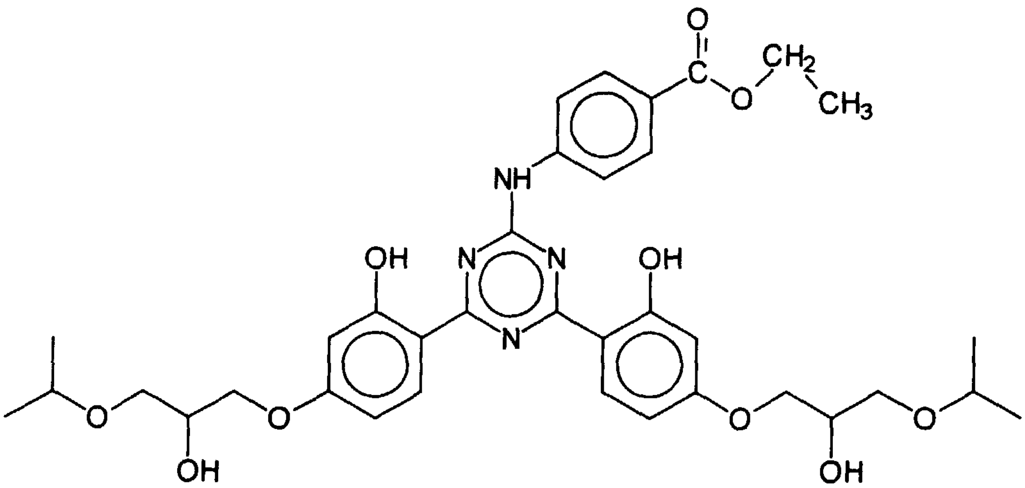

Ferner vorteilhaft ist das 2,4-Bis-{[4-(2-ethyl-hexyloxy)-2-hydroxy]-phenyl}-6-(1-methylpyrrol-2-yl)-1,3,5-triazin, welches durch folgende Struktur gekennzeichnet ist:

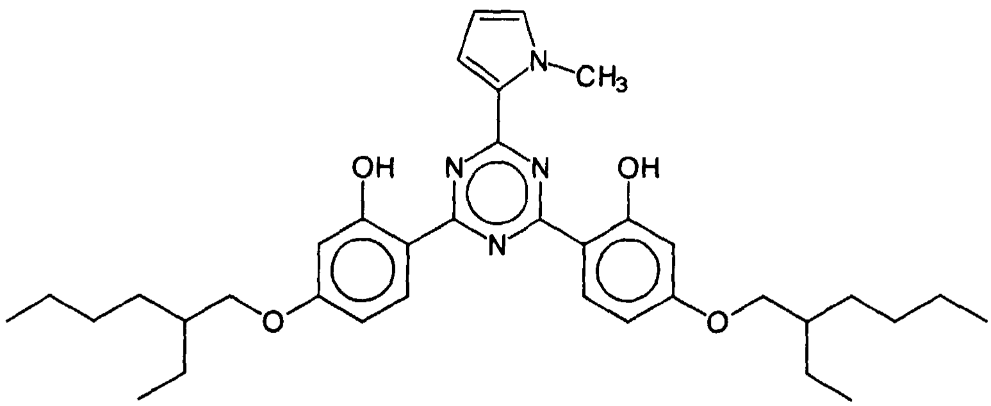

Ferner vorteilhaft ist das 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist:

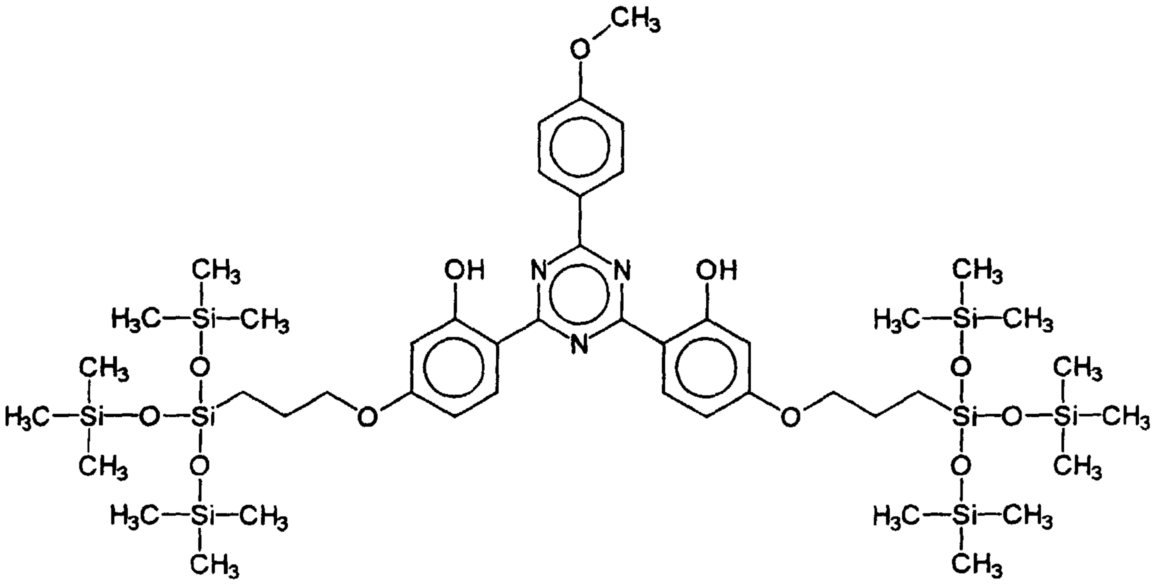

Ferner vorteilhaft ist das 2,4-Bis-{[4-(2 "-methylpropenyloxy)-2-hydroxy]-phenyl}-6-(4-methoxyphenyl)-1,3,5-triazin, welches durch folgende Struktur gekennzeichnet ist:

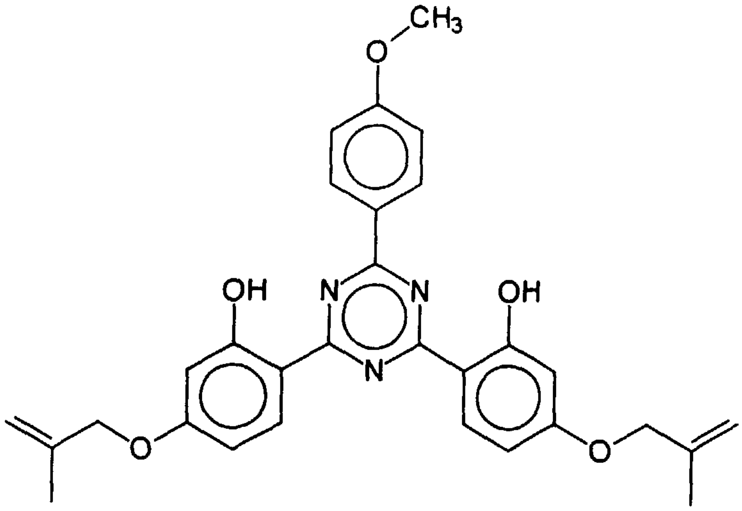

Ferner vorteilhaft ist das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin, welches durch folgende Struktur gekennzeichnet ist:

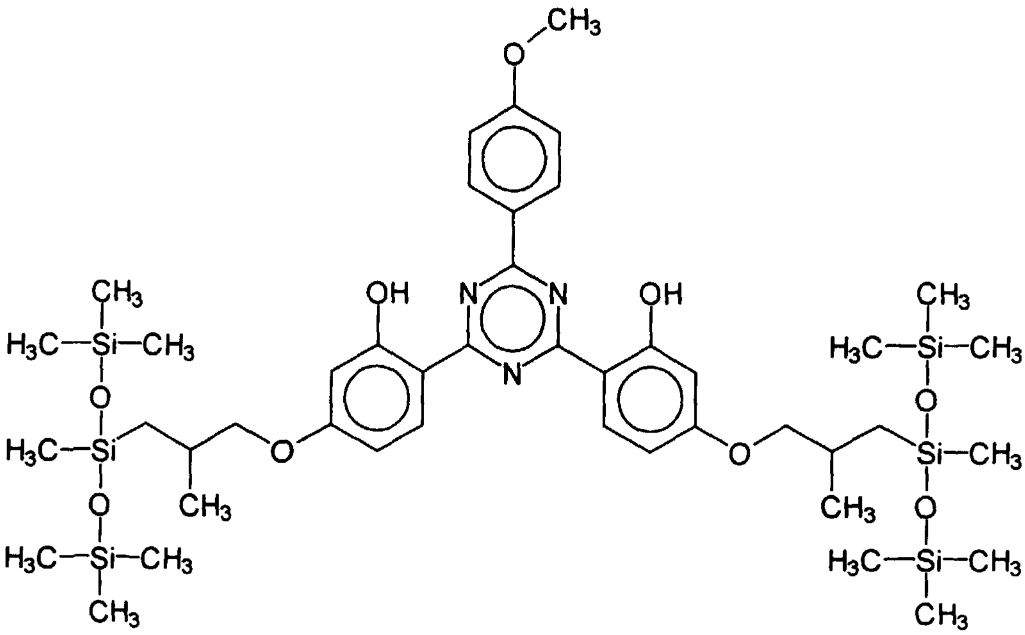

In einer besonders bevorzugten Ausführungsform betrifft die vorliegende Erfindung die Verwendung eines asymmetrisch substituierten s-Triazins, dessen chemische Struktur durch die Formel

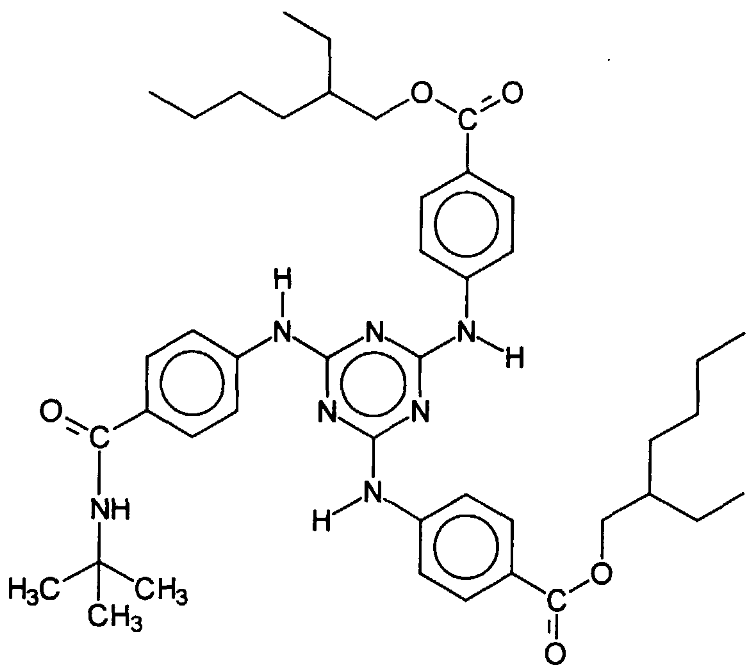

wiedergegeben wird (Dioctylbutamidotriazon).

Bei erfindungsgemäßer Verwendung eines oder mehrerer eines oder mehrerer aus den Amidverbindungen ausgewählter Öle haben eine oder mehrere relativ schwerlösliche Komponenten, gewählt aus der Gruppe der unsymmetrisch substituierten s-Triazindenvate, eine bedeutend bessere Löslichkeit in

(a) entweder einer isolierten Ölkomponente oder
(b) in mindestens einer Ölphase eines dispersen Zwei- oder Mehrphasensystem welches zusätzlich eine oder mehrere Wasserphasen enthalten kann,

als in den Zubereitungen des Standes der Technik.

Es ist erfindungsgemäß bevorzugt, die dispersen Zwei- oder Mehrphasensysteme, welche zusätzlich eine oder mehrere Wasserphasen enthalten können, in Form kosmetischer oder dermatologischer Emulsionen, beispielsweise vom Typ W/O, O/W, W/O/W oder O/W/O auszugestalten.

Selbst wenn als eine der Ölkomponenten, als die überwiegende Ölkomponente oder als die einzige Ölkomponente, sei es in isolierter Form oder in mindestens einer Ölphase eines dispersen Zwei- oder Mehrphasensystems, welches zusätzlich eine oder mehrere Wasserphasen enthalten kann, der Dicaprylylether oder vergleichbare Substanzen gewählt wird oder werden, ist die Löslichkeit von unsymmetrisch substituierten s-Triazinen in den betreffenden Systemen erheblich erhöht.

Ferner sind erfindungsgemäß Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Weiterhin sind erfindungsgemäß besonders gut hautverträgliche Zubereitungen erhältlich.

Voraussetzung für die Verwendbarkeit der erfindungsgemäßen Wirkstoffkombinationen für die erfindungsgemäßen Zwecke ist natürlich die kosmetische bzw. dermatologische Unbedenklichkeit der zugrundeliegenden Substanzen.

Es ist erfindungsgemäß möglich die Einsatzmengen von unsymmetrisch substituierten s-Triazinen, insbesondere vom Dioctylbutamidotriazon in kosmetischen oder dermatologischen Zubereitungen gegenüber dem Stande der Technik beispielsweise zu verdoppeln.

Ferner war erstaunlich, daß durch Zugabe eines oder mehrerer aus den Amidverbindungen ausgewählter Öle eine Stabilisierung von Lösungen von einem oder mehreren symmetrisch substituierten s-Triazinderivaten, insbesondere vom Dioctylbutamidotriazon in

(a) entweder einer isolierten Ölkomponente oder
(b) in mindestens einer Ölphase eines dispersen Zwei- oder Mehrphasensystems, welches zusätzlich eine oder mehrere Wasserphasen enthalten kann,

bewirkt wird.

Die Gesamtmenge an einem oder mehreren unsymmetrisch substituierten s-Triazindenvaten, insbesondere von Dioctylbutylamidotriazon, in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen.

Es ist erfindungsgemäß bevorzugt, die Gewichtsverhältnisse zwischen einem oder mehreren unsymmetrisch substituierten s-Triazinderivaten, insbesondere von Dioctylbutylamidotriazon, und einem oder mehreren aus den Amidverbindungen ausgewählten Ölen aus dem Bereich von 1 : 8 bis 8 : 1, bevorzugt 1 : 4 bis 4 : 1, insbesondere bevorzugt 1 : 2 bis 2 : 1, zu wählen.

Es ist erfindungsgemäß auch gegebenenfalls vorteilhaft, zusätzlich ein oder mehrere symmetrisch substituierte Triazinderivate, insbesondere 4,4',4"-(1,3,5-Triazin-2,4,6-triyltriimino)-tris-benzoesäure-tris(2-ethylhexylester), einzusetzen, wobei die Gewichtsverhältnisse zwischen einem oder mehreren symmetrisch substituierten s-Triazinderivaten, insbesondere von 4,4',4"-(1,3,5-Triazin-2,4,6-triyltriimino)-tris-benzoesäure-tris-(2-ethylhexylester) und einem oder mehreren unsymmetrisch substituierten s-Triazinderivaten, insbesondere von Dioctylbutylamidotriazon, aus dem Bereich von 1 : 8 bis 4 : 1, bevorzugt 1 : 4 bis 2 : 1, insbesondere bevorzugt 1 : 2 bis 1 : 1, zu wählen.

Kosmetische und dermatologische Zubereitungen gemäß der Erfindung enthalten vorteilhaft, wenngleich nicht zwingend, anorganische Pigmente, welche röntgenamorph oder nichtröntgenamorph sind, auf Basis von Metalloxiden und/oder anderen in Wasser schwerlöslichen oder unlöslichen Metailverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Räntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nichtröntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handeisbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung,

der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen gemäß der Erfindung können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonäle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylberizoat und Isotridecylisononanoat sowie Mischungen aus $C_{12-15}$-Alkylbenzoat, 2-Ethylhexylisostearat und Isotndecylisononanoat.

Von den Kohlenwasserstoffen sind Paraffinöl, Squalan und Squalen vorteilhaft im Sinne der vorhegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecytisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäße, als Emulsionen vorliegende Zubereitungen enthalten einen oder mehrere Emulgatoren. O/W-EMulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel $R-O-(-CH_2-CH_2-O-)_n-R'$,
- der Fettsäureethoxylate der allgemeinen Formel $R-COO-(-CH_2-CH_2-O-)_n-H$,
- der veretherten Fettsäureethoxylate der allgemeinen Formel $R-COO-(-CH_2-CH_2-O-)_n-R'$,
- der veresterten Fettsäureethoxylate der allgemeinen Formel $R-COO(-CH_2-CH_2O-)_n-C(O)R'$,
- der Polyethylenglycolglycerinfettsäureester
- der ethoxylierten Sorbitanester

- der Cholesterinethoxylate
- der ethoxylierten Triglyceride
- der Alkylethercarbonsäuren der allgemeinen Formel R-O-(-$CH_2$-$CH_2$-O-)$_n$-$CH_2$-COOH und n eine Zahl von 5 bis 30 darstellen,
- der Polyoxyethylensorbitolfettsäureester,
- der Alkylethersulfate der allgemeinen Formel R-O-(-$CH_2$-$CH_2$-O-)$_n$-$SO_3$-H
- der Fettalkoholpropoxylate der allgemeinen Formel R-O-(-$CH_2$-CH($CH_3$)-O-)$_n$-H,
- der Polypropylenglycolether der allgemeinen Formel R-O-(-$CH_2$-CH($CH_3$)-O-)$_n$-R',
- der propoxylierten Wollwachsalkohole,
- der veretherten Fettsäurepropoxylate R-COO-(-$CH_2$-CH($CH_3$)-O-)$_n$-R',
- der veresterten Fettsäurepropoxylate der allgemeinen Formel R-COO-(-$CH_2$-CH($CH_3$)-O-)$_n$-C(O)-R',
- der Fettsäurepropoxylate der allgemeinen Formel R-COO-(-$CH_2$-CH($CH_3$)-O-)$_n$-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-$CH_2$-CH($CH_3$)O-)$_n$-$CH_2$-COOH
- der Alkylethersulfate bzw. die diesen Sulfaten zugrundeliegenden Säuren der allgemeinen Formel R-O-(-$CH_2$-CH($CH_3$)-O-)$_n$-$SO_3$-H
- der Fettalkoholethoxylate/propoxylate der allgemeinen Formel R-O-$X_n$-$Y_m$-H,
- der Polypropylenglycolether der allgemeinen Formel R-O-$X_n$-$Y_m$-R',
- der veretherten Fettsäurepropoxylate der allgemeinen Formel R-COO-$X_n$-$Y_m$-R',
- der Fettsäureethoxylate/propoxylate der allgemeinen Formel R-COO-$X_n$-$Y_m$-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)-stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlange von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bomylidenmethyl)benzolsulfonsäure,2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtem zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert. Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Beispiel 1

| O/W-Emulsion | Gew.-% |
|---|---|
| Stearinsäure | 1,50 |
| Glycerinmonostearat | 3,00 |
| Dimethicon | 2,00 |
| Vitamin E-Acetat | 0,50 |
| Aniso Triazin | 4,00 |
| Repellent 3535 ® | 10,00 |
| Glycerin | 3,00 |
| Xanthan Gummi | 0,30 |
| Natronlauge 45% | 0,50 |
| Konservierung, Parfum, Farbstoffe | q.s. |
| Wasser | ad 100,00 |

Beispiel 2

| O/W-Emulsion | Gew.-% |
|---|---|
| Stearinsäure | 1,50 |
| Glycerinmonostearat | 3,00 |
| Caprylsäure/Caprinsäuretriglyceride | 5,00 |
| Dicaprylylether | 5,00 |
| Dimethicon | 1,00 |
| Butylenglycoldicaprylat/caprat | 2,00 |
| $C_{12-15}$ Alkylbenzoate | 3,00 |
| Vitamin E-Acetat | 0,50 |
| Dioctylbutamidotriazon | 2,00 |
| Aniso Triazin | 2,00 |
| Repellent 3535 ® | 5,00 |
| Octyltriazon | 1,00 |
| Methylbenzylidencampher | 4,00 |
| Butylmethoxydibenzoylmethan | 2,00 |
| Titandioxid | 1,00 |
| Glycerin | 5,00 |
| Pemulen TR1 ® | 0,20 |
| Natronlauge 45% | 0,70 |
| Konservierung, Parfum, Farbstoffe | q.s. |
| Wasser | ad 100,00 |

Beispiel 3

| O/W-Emulsion | Gew.-% |
|---|---|
| Sorbitanstearat | 3,00 |
| Polyglyceryl-3 Methylglucosedistearat | 1,50 |
| Octyldodecanol | 10,00 |
| Dicaprylylether | 5,00 |
| Cetylstearylisononanoat | 2,00 |
| Butylenglycoldicaprylat/caprat | 5,00 |
| Vitamin E-Acetat | 0,50 |
| Dioctylbutamidotriazon | 6,00 |

(fortgesetzt)

| O/W-Emulsion | Gew.-% |
|---|---|
| Repellent 3535 ® | 10,00 |
| Octyltriazon | 4,00 |
| Butylmethoxydibenzoylmethan | 3,00 |
| Glycerin | 10,00 |
| Xanthan Gummi | 0,20 |
| Pemulen TR1 ® | 0,10 |
| Phenylbenzimidazolsulfonsäure | 2,00 |
| Natronlauge 45% | 1,20 |
| Konservierung, Parfum, Farbstoffe | q.s. |
| Wasser | ad 100,00 |

Beispiel 4

| W/O-Emulsion | Gew.-% |
|---|---|
| Polyglyceryl-2 dipolyhydroxystearat | 5,00 |
| Dimethicon | 2,00 |
| Mineralöl | 5,00 |
| Isohexadecan | 5,00 |
| Butylenglycoldicaprylat/caprat | 5,00 |
| Dioctylbutamidotriazon | 3,00 |
| Aniso Triazin | 1,00 |
| Repellent 3535 ® | 8,00 |
| Titandioxid | 2,00 |
| Glycerin | 5,00 |
| $MgSO_4$ | 1,00 |
| Konservierung, Parfum, Farbstoffe | q. s. |
| Wasser | ad 100,00 |

Beispiel 5

| W/O-Emulsion | Gew.-% |
|---|---|
| PEG-30-dipolyhydroxystearat | 4,00 |
| Caprylsäure/Caprinsäuretriglyce-ride | 5,00 |

(fortgesetzt)

| W/O-Emulsion | Gew.-% |
|---|---|
| Octyldodecanol | 5,00 |
| Dicaprylylether | 5,00 |
| Mineralöl | 5,00 |
| Isohexadecan | 5,00 |
| Vitamin E-Acetat | 0,50 |
| Dioctylbutamidotriazon | 5,00 |
| Repellent 3535 ® | 15,00 |
| Aerosil R972 ® | 0,50 |
| Glycerin | 10,00 |
| $MgSO_4$ | 1,00 |
| Konservierung, Parfum, Farbstoffe | q.s. |
| Wasser | ad 100,00 |

Beispiel 6

| W/O-Emulsion | Gew.-% |
|---|---|
| Cetyldimethiconcopolyol | 5,00 |
| Dimethicon | 5,00 |
| Isohexadecan | 2,00 |
| Butylenglycoldicaprylat/caprat | 8,00 |
| $C_{12-15}$ Alkylbenzoate | 5,00 |
| Dioctylbutamidotriazon | 2,00 |
| Aniso Triazin | 2,00 |
| Repellent 3535 ® | 10,00 |
| Octyltriazon | 1,00 |
| Methylbenzylidencampher | 4,00 |
| Butylmethoxydibenzoylmethan | 2,00 |
| Titandioxid | 2,00 |
| Glycerin | 5,00 |
| NaCl | 1,00 |
| Phenylbenzimidazolsulfonsäure | 4,00 |
| Natronlauge 45% | 1,30 |
| Konservierung, Parfum, Farbstoffe | q.s. |
| Wasser | ad 100,00 |

Beispiel 7

| Spray | Gew.-% |
|---|---|
| Glycerinmonostearat | 4,00 |
| Ceteareth-12 | 1,50 |
| Caprylsäure/Caprinsäuretriglyceride | 2,00 |
| Mineralöl | 5,00 |
| Dioctylbutamidotriazon | 0,50 |
| Repellent 3535 ® | 2,00 |
| Octyltriazon | 1,00 |
| Butylmethoxydibenzoylmethan | 1,00 |
| Glycerin | 10,00 |
| Phenylbenzimidazolsulfonsäure | 1,00 |
| Natronlauge 45% | 0,40 |
| Konservierung, Parfum, Farbstoffe | q.s. |
| Wasser | ad 100,00 |

Beispiel 8

| Spray | Gew.-% |
|---|---|
| Glycerinmonostearat SE | 4,50 |
| Ceteareth-20 | 1,00 |
| Dicaprylylether | 5,00 |
| Cetylstearylisononanoat | 5,00 |
| Dimethicon | 2,00 |
| Dioctylbutamidotriazon | 1,00 |
| Aniso Triazin | 1,00 |
| Repellent 3535 ® | 5,00 |
| Glycerin | 5,00 |
| Konservierung, Parfum, Farbstoffe | q.s. |
| Wasser | ad 100,00 |

## Patentansprüche

1. Kosmetische Lichtschutzzubereitungen, enthaltend ein oder mehrere aus den Amidverbindungen ausgewählte Öle und ein oder mehrere unsymmetrisch substituierte Triazinderivate.

**2.** Lichtschutzzubereitungen nach Anspruch 1, dadurch gekennzeichnet, daß das oder die aus den Amidverbindungen ausgewählten Öle gewählt werden aus der Gruppe der Substanzen der generischen Strukturformel

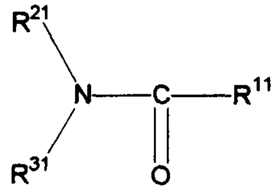

,

in welcher $R^{11}$, $R^{21}$ und $R^{31}$ unabhängig voneinander gewählt werden aus der Gruppe der verzweigten und unverzweigten, gesättigten und ungesättigten und gegebenenfalls funktional substituierten Alkylreste und/oder Cycloalkyreste und/oder gegebenenfalls funktional substituierten Arylreste, und wobei $R^{21}$ und/oder $R^{31}$ auch die Struktur

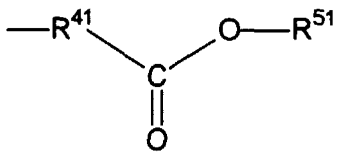

annehmen kann oder können, in weicher $R^{41}$ eine Alkylengruppe mit bis zu 10 Kohlenstoffatome bedeutet und $R^{51}$ gewählt wird aus der Gruppe der verzweigten und unverzweigten, gesättigten und ungesättigten und gegebenenfalls funktional substituierten Alkylreste.

**3.** Lichtschutzzubereitungen nach Anspruch 1 oder 2, dadurch gekennzeichnet, daß das oder die aus den Amidverbindungen ausgewählten Öle gewählt werden aus der Gruppe 3-(N-Acetyl-N-butylamino)-propionsäureethylester, Octansäurediethylamid, N,N-Diethyl-m-toluamid

**4.** Lichtschutzzubereitungen nach einem der Ansprüche 1 - 3, dadurch gekennzeichnet, daß die chemische Struktur des oder der unsymmetrisch substituierten s-Triazinderivate, durch die generische Formel

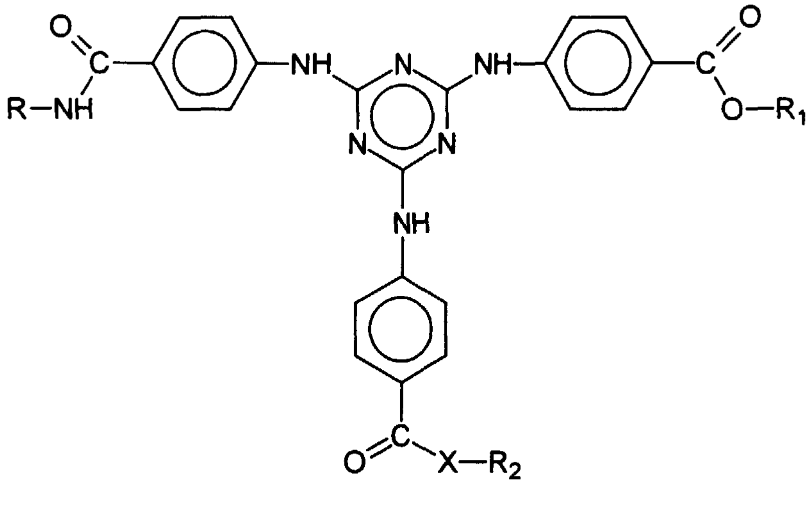

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
X ein Sauerstoffatom oder eine NH-Gruppe darstellt,
$R_1$ einen verzweigten oder unverzweigten $C_1$-$C_{18}$-Alkylrest, einen $C_5$-$C_{12}$-Cycloalkylrest, gegebenenfalls sub-

stituiert mit einer oder mehreren $C_1$-$C_4$- Alkylgruppen, oder ein Wasserstoffatom, ein Alkalimetallatom, eine Ammoniumgruppe oder eine Gruppe der Formel

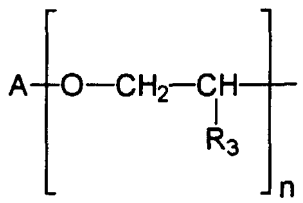

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

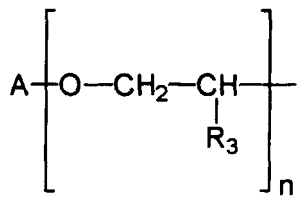

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

**5.** Lichtschutzzubereitungen nach einem der Ansprüche 1 - 4, dadurch gekennzeichnet, daß als unsymmetrisch substituiertes s-Triazinderivat das Dioctylbutamidotriazon und/oder das 2,4-Bis-{[4-(2-Ethyl-hexyloxy)-2-hydroxy]-phenyl}-6-(4-methoxyphenyl)-1,3,5-triazin gewählt wird.

**6.** Lichtschutzzubereitungen nach einem der Ansprüche 1 - 5, dadurch gekennzeichnet, daß die Gesamtmenge an einem oder mehreren unsymmetrisch substituierten s-Triazinderivaten, insbesondere von Dioctylbutylamidotriazon und/oder 2,4-Bis-{[4-(2-Ethyl-hexyloxy)-2-hydroxy]-phenyl}-6-(4-methoxyphenyl)-1,3,5-triazin, in den fertigen kosmetischen oder dermatologischen Zubereitungen vorteilhaft aus dem Bereich von 0,1 - 15,0 Gew.-%, bevorzugt 0,5 - 8,0 Gew.-% gewählt wird, bezogen auf das Gesamtgewicht der Zubereitungen.

**7.** Lichtschutzzubereitungen nach einem der Ansprüche 1 - 6, dadurch gekennzeichnet, daß die Gesamtmenge an einem oder mehreren aus den Amidverbindungen ausgewählten Öle in den fertigen kosmetischen oder dermatologischen Zubereitungen aus dem Bereich von 0,1 - 15,0 Gew.-% gewählt wird, bevorzugt 0,5 - 8,0 Gew.-%, bezogen auf das Gesamtgewicht der Zubereitungen.

8. Lichtschutzzubereitungen nach einem der Ansprüche 1 - 7, dadurch gekennzeichnet, daß Gewichtsverhältnisse zwischen einem oder mehreren unsymmetrisch substituierten s-Triazinderivaten, insbesondere von Dioctylbutylamidotriazon, und einem oder mehreren aus den Amidverbindungen ausgewählten Ölen aus dem Bereich von 1 : 8 bis 8 : 1, bevorzugt 1 : 4 bis 4 : 1, insbesondere bevorzugt 1 : 2 bis 2 : 1, gewählt werden.